# EUROPEAN PATENT SPECIFICATION

(11) **EP 3 181 035 B1**
(45) Date of publication and mention of the grant of the patent: **13.09.2023**
(21) Application number: 15831728.9
(22) Date of filing: 11.08.2015
(51) Int. Cl.: A61B 1/00, G02B 6/38, A61B 5/00, A61B 8/00, A61B 8/12, G02B 23/24, G02B 23/26

(54) **CONNECTION DEVICE AND IMAGING DEVICE PROVIDED WITH SAID CONNECTION DEVICE**
VERBINDUNGSVORRICHTUNG UND MIT DER BESAGTEN VERBINDUNGSVORRICHTUNG AUSGESTATTETE BILDGEBUNGSVORRICHTUNG
DISPOSITIF DE CONNEXION ET DISPOSITIF D'IMAGERIE ÉQUIPÉ DUDIT DISPOSITIF DE CONNEXION

(30) Priority: 14.08.2014 JP 2014165228
(43) Date of publication of application: 21.06.2017
(73) Proprietor: Terumo Kabushiki Kaisha, Tokyo 151-0072 (JP)
(72) Inventor: HORIIKE, Toyokazu, Fujinomiya-shi Shizuoka 418-0015 (JP)
(74) Representative: Casalonga
(86) International application number: PCT/JP2015/072764
(87) International publication number: WO 2016/024588

(56) References cited:
- JP-A- 2000 138 471
- JP-A- 2010 057 895
- JP-A- 2014 074 809
- JP-B2- 5 139 298
- JP-B2- 5 150 725
- JP-U- 3 179 931
- US-A- 4 634 214
- US-A1- 2012 002 928
- US-A1- 2012 226 151

## Description

### Technical Field

The present invention relates to a connection device for connecting information transmission cables to each other, and an imaging apparatus provided with the connection device.

### Background Art

US 2012/002928 A1 relates to a connection device according to the preamble of claim 1.

US4634214 relates to an optical fiber connector comprising a fiber terminus piece with a longitudinal bore through which a bare fiber is inserted.

Recently, as the large-capacity communication and the like advance, mainly in the telecommunications field, a connection device for optical fiber cables has been adopted in order to connect optical fiber cables to each other.

In the medical field, various types of medical instruments such as an optical coherence tomography apparatus (OCT) are used in order to perform medical diagnosis or treatment utilizing light. In these medical instruments, for example, light is utilized as a signal, and the optical fiber cables are used when transmitting an optical signal. Therefore, in the field of medical instruments, the connection device is inevitably used in order to connect the optical fiber cables to each other.

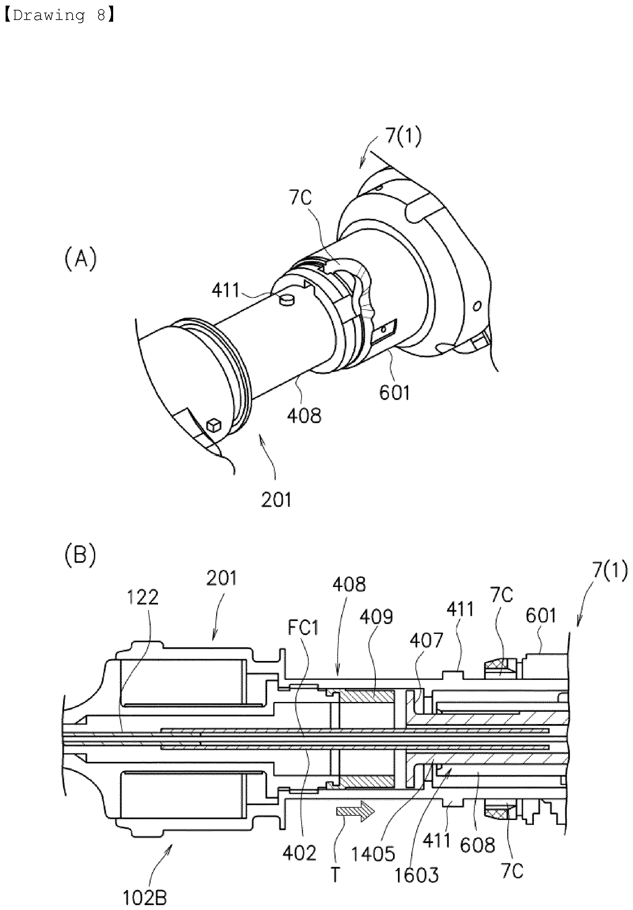

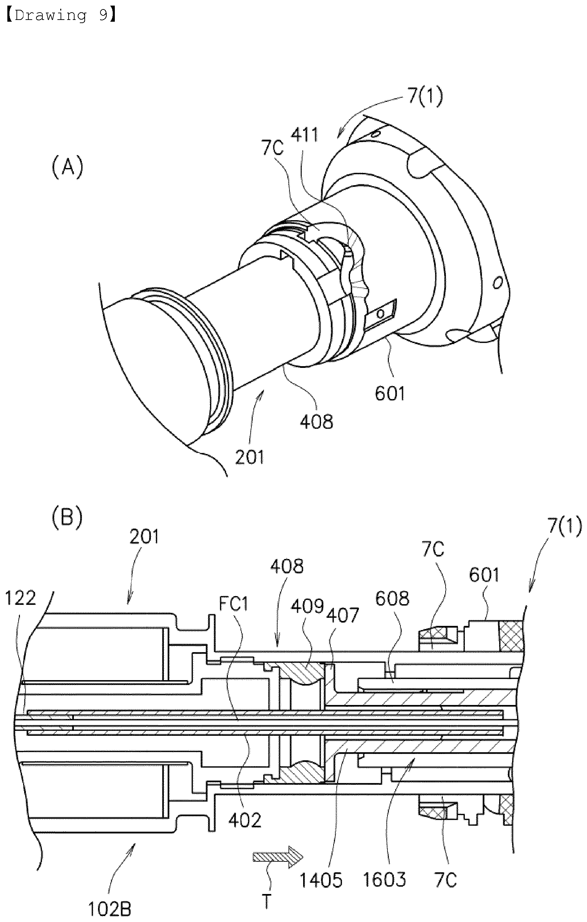

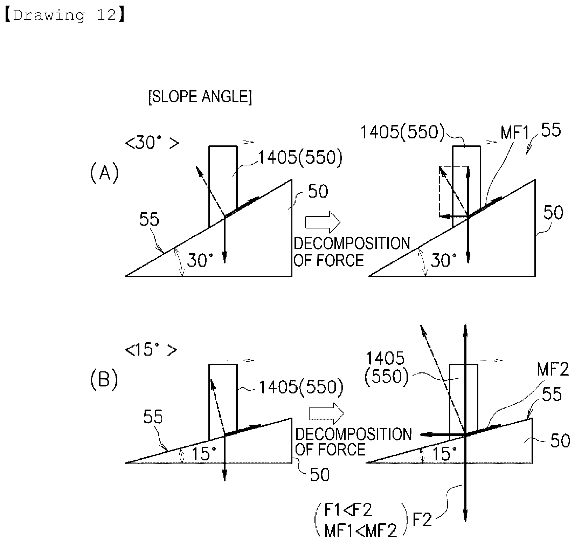

PTL 1 discloses an optical fiber cable connection device disposed in an optical imaging apparatus. As illustrated in Fig. 12 of PTL 1, the connection device includes a connector member 405 that is connected to a catheter side, and an adapter fixing member 603 inside a motor drive unit (MDU). The connector member 405 to be connected to the catheter connects end portions of optical fiber cables to each other by being inserted into the adapter fixing member 603. As illustrated in Fig. 9 of PTL 1, the connector member 405 has a protruding portion 702 on an outer peripheral surface. In addition, as illustrated in Fig. 8, a guide tube 609 inside the adapter fixing member 603 has slope end surfaces 801A and 801B respectively in two directions bifurcated from the vertex, and a cut-off portion 802.

As illustrated in Fig. 12, when the connector fixing member 405 is inserted into the adapter fixing member 603 and the end portions of the optical fiber cables are connected to each other, the protruding portion 702 of the connector fixing member 405 is guided in a manner of gyrating along any one of the slope end surfaces 801A and 801B of the adapter fixing member 603. Accordingly, the connector fixing member 405 and the adapter fixing member 603 relatively rotate, and the protruding portion 702 of the connector fixing member 405 is fitted into the cut-off portion 802 of the adapter fixing member 603.

In this manner, when the connector fixing member 405 and the adapter fixing member 603 are connected to each other, the end portion of the optical fiber cable inside the connector fixing member 405 and the end portion of the optical fiber cable inside the adapter fixing member 603 are connected to each other.

### Citation List

### Patent Literature

[PTL 1] U.S. Patent No. 8322932

### Summary of Invention

### Technical Problem

In a connection device disclosed in PTL 1 described above, when a connector fixing member 405 is intended to be inserted into an adapter fixing member 603 such that the connector fixing member 405 is connected to the adapter fixing member 603, a protruding portion 702 of the connector fixing member 405 can gyrate while following any one of slope end surfaces 801A and 801B respectively in two directions.

Therefore, there is concern that the protruding portion 702 of the connector fixing member 405 bumps into a sharp distal portion of the slope end surfaces 801A and 801B respectively in two directions, is drawn into an inner surface side of a guide tube 609 of the adapter fixing member 603, and causes jamming between the protruding portion 702 of the connector fixing member 405 and the adapter fixing member 603.

Therefore, as a result of earnest endeavor and studies carried out by the inventor so as to prevent jamming, the present invention has been made and an object thereof is to provide a connection device in which information transmission cables can be reliably connected to each other while jamming is prevented from occurring in the connection device, and an imaging apparatus provided with the connection device.

### Solution to Problem

Hereinafter, description will be given regarding specified configurations of the present invention and operation effects which are realized based on each of the configurations. In this case, in order to make the description easy to understand, the reference signs used for illustrating the below-described embodiments are applied to the corresponding configurations. However, the present invention is not limited to the detailed configurations of the embodiments to which the reference signs are applied.

According to the present invention, there is provided a connection device (200) according to claim 1. Preferred embodiments are disclosed in the dependent claims.

In the configuration, in the connection device according to the present invention, when the connector fixing member (1405) is inserted into the guide member (50) in order to connect the end portion of the first information transmission cable (FC1) and the end portion of the second information transmission cable (FC2) to each other, the projection portion (550) of the connector fixing member (1405) can be guided in a manner of gyrating along the helical guide following surface (55) formed in one direction in the guide member (50) without confusing a gyratory direction. That is, the projection portion (550) of the connector fixing member (1405) can be reliably guided in a manner of gyrating in only one direction along the helical guide following surface (55) formed in one direction in the guide member (50). Accordingly, in the present invention, the connector fixing member (1405) and the guide member (50) can be smoothly coupled to each other, and the information transmission cables can be reliably connected to each other while jamming is prevented from occurring in the connection device.

In contrast, in a case of a structure in the related art, the connector fixing member (405) gyrates in any direction of slope end surfaces (801A, 801B) respectively in two directions. Since there are two slope surfaces, the angle of a sharp distal portion is significant, and there is concern that jamming may occur in the connection device.

It is preferable that an inner surface of the adapter fixing member (1603) is provided with a slope restraining projection portion (880) which restrains the connector fixing member (1405) from sloping with respect to the adapter fixing member (1603) when the connector fixing member (1405) is inserted into the guide member (50).

In the configuration, even though a force that tends to cause the connector fixing member (1405) to slope tends to be generated when the connector fixing member (1405) is intended to be inserted in the inner surface of the adapter fixing member (1603), the projection portion (880) causes a force that restrains sloping to act on the connector fixing member (1405) . Therefore, the projection portion (880) restrains a force that tends to cause the connector fixing member (1405) to slope from being generated, and thus, it is possible to further prevent a phenomenon in which the projection portion (550) of the connector fixing member (1405) is drawn into an inner surface of the guide member (50) and causes jamming therein.

It is preferable that an outer peripheral surface of the connection section (201) is provided with a projection portion (411). It is preferable that the connection device (200) further includes a connection holding body (7) that internally accommodates the adapter fixing member (1603). It is preferable that the connection holding body (7) is provided with a helical guide groove portion (910) which guides the projection portion (411) in a manner of gyrating around the central axis (CL) . It is preferable that before an operation in which the projection portion (550) of the connector fixing member (1405) is guided by the helical guide following surface (55) in a gyrating manner starts, an operation in which the projection portion (411) of the connection section (201) is guided by the helical guide groove portion (910) in a gyrating manner is configured to start.

In the configuration, at the point of time the projection portion (411) of the connection section (201) enters the helical guide groove portion (910), the projection portion (550) of the connector fixing member (1405) has not yet bumped into a helical vertex portion of the helical guide following surface (55) of the guide member (50), and a gyration guiding operation for the projection portion (411) of the connection section (201) is performed prior thereto along the helical guide groove portion (910) from a leading end portion of the helical guide groove portion (910) and proceeds. When the projection portion (550) hits the helical guide following surface (55) of the guide member (50), the projection portion (550) is guided by the helical guide following surface (55) in a gyrating manner. Accordingly, the projection portion (550) can be smoothly guided by the helical guide following surface (55), and the end portions of the information transmission cables can be reliably and smoothly connected to each other.

It is preferable that a distal portion on the outer peripheral surface of the connector fixing member (1405) is provided with a guide projection (890) which guides the connector fixing member (1405) in a manner of gyrating around the central axis (CL) by coming into contact with the helical guide following surface (55) of the guide member (50) prior to the projection portion (550) when the connector fixing member (1405) is inserted into the guide member (50).

In the configuration, when the connector fixing member (1405) is inserted, the guide projection (890) hits the helical vertex portion of the helical guide following surface (55) prior to the projection portion (550). Therefore, it is possible to induce rotation along the helical guide following surface (55) of the guide member (50). That is, before the guiding projection portion (550) hits the helical vertex portion, the guide projection (890) bumps into the helical vertex portion. Therefore, the projection portion (550) can avoid hitting the helical vertex portion. Accordingly, the projection portion (550) can be caused to smoothly copy the helical guide following surface (55) from the position where the projection portion (550) has avoided the helical vertex portion of the helical guide following surface (55) .

It is preferable that the first information transmission cable has an imaging core, and the imaging core generates an optical signal or an acoustic signal.

In the configuration, the imaging core can adopt any one of the optical signal and the acoustic signal in order to form an image.

There is provided an imaging apparatus (500) provided with the connection device according to the present invention. The imaging apparatus (500) includes a catheter unit (100) that has a connection section (201); a drive device (1) that has a connection holding body (7), emits a signal to a target through a first information transmission cable (FC1) and a second information transmission cable (FC2), and obtains a signal from the target through the first information transmission cable (FC1) and the second information transmission cable (FC2); and an operation control apparatus (501) that forms an image of the target in accordance with a signal which is generated based on the signal from the target and is sent from the drive device (1).

In the configuration, in the imaging apparatus provided with the connection device according to the present invention, when the connector fixing member (1405) is inserted into the guide member (50) in order to connect the end portion of the first information transmission cable (FC1) and the end portion of the second information transmission cable (FC2) to each other, the projection portion (550) of the connector fixing member (1405) can be guided in a manner of gyrating along the helical guide following surface (55) formed in one direction in the guide member (50) without confusing a gyratory direction. That is, the projection portion (550) of the connector fixing member (1405) can be reliably guided in a manner of gyrating in only one direction along the helical guide following surface (55) formed in one direction in the guide member (50). Accordingly, in the present invention, the connector fixing member (1405) and the guide member (50) can be smoothly coupled to each other, and the information transmission cables can be reliably connected to each other while jamming is prevented from occurring in the connection device.

### Advantageous Effect of Invention

According to the present invention, it is possible to provide the connection device in which the information transmission cables can be reliably connected to each other while jamming is prevented from occurring in the connection device, and the imaging apparatus provided with the connection device.

### Brief Description of Drawings

[Fig. 1] Fig. 1 is a perspective view illustrating the appearance of an optical coherence tomography apparatus which serves as an imaging apparatus and is provided with a connection device of a first embodiment of the present invention.
[Fig. 2] Fig. 2 is a perspective view illustrating the appearance of a scanner & pull-back unit (motor drive unit: MDU) illustrated in Fig. 1.
[Fig. 3] Fig. 3 is a view illustrating a structural example of a catheter unit illustrated in Fig. 1.
[Fig. 4] Fig. 4 is a cross-sectional view illustrating a structural example of a regional portion BB of a connection section of a drive shaft connector and a regional portion CC of a catheter connection section of the scanner & pull-back unit illustrated in Fig. 3.
[Fig. 5] Fig. 5 is a view illustrating an example of a shape of a connector fixing member.
[Fig. 6] Fig. 6 is a perspective view illustrating the connector fixing member illustrated in Fig. 5, and a guide member of an adapter fixing member.
[Fig. 7] Fig. 7 is a view illustrating a state where the guide member of the adapter fixing member relatively rotates.
[Fig. 8] Fig. 8 is a view illustrating a structural example of connecting and fixing the connection section of the drive shaft connector and the catheter connection section of the scanner & pull-back unit to each other not forming part of the invention, and the view illustrates a state immediately before being fixed.
[Fig. 9] Fig. 9 is a view illustrating a state in the middle of an operation of fixing the connection section of the drive shaft connector and the catheter connection section of the scanner & pull-back unit to each other not forming part of the invention.
[Fig. 10] Fig. 10 is a view illustrating a state where the connection section of the drive shaft connector and the catheter connection section of the scanner & pull-back unit are fixed to each other not forming part of the invention.
[Fig. 11] Fig. 11 is a view illustrating the first embodiment of the present invention and a comparative example not forming part of the invention which are contrasted with each other.
[Fig. 12] Fig. 12 is a view describing a relationship of the first embodiment of the present invention with respect to that in Fig. 11.
[Fig. 13] Fig. 13 is a perspective view illustrating a second embodiment of the present invention.
[Fig. 14] Fig. 14 is a side view including a cross section illustrating the second embodiment of the present invention.
[Fig. 15] Fig. 15 is a perspective view illustrating a third embodiment not forming part of the present invention.
[Fig. 16] Fig. 16 is a cross-sectional view illustrating the third embodiment not forming part of the present invention.
[Fig. 17] Fig. 17 is a perspective view illustrating a fourth embodiment of the present invention.
[Fig. 18] Fig. 18 is a side view illustrating the fourth embodiment of the present invention.
[Fig. 19] Fig. 19 is a cross-sectional view illustrating a fifth embodiment not forming part of the present invention.

### Description of Embodiments

Hereinafter, preferable embodiments of the present invention will be described in detail with reference to the drawings.

Note that, the embodiments described below are specific examples suitable for the present invention, and various types of technically preferable limitation are applied to the embodiments. However, the scope of the present invention is defined in the claims.

### (First Embodiment)

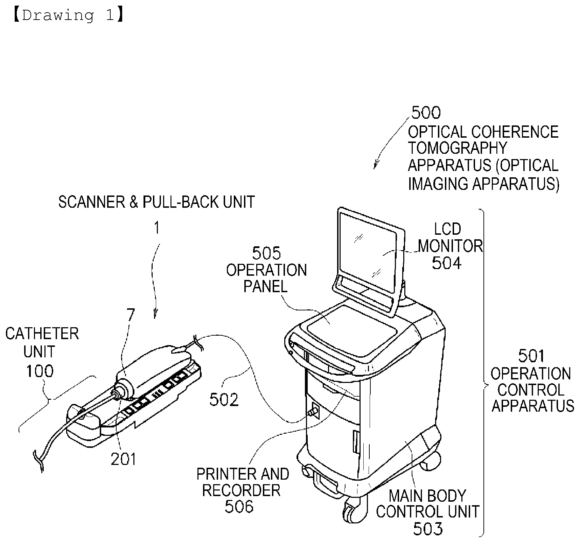

Fig. 1 is a perspective view illustrating the appearance of an optical coherence tomography apparatus 500 provided with a connection device of a first embodiment of the present invention. The optical coherence tomography apparatus 500 is an example, particularly an optical imaging apparatus among imaging apparatuses.

However, the imaging apparatus according to the present invention is not limited to the optical coherence tomography apparatus 500 which forms an image by using optical signals. The imaging apparatus may be a different medical instrument, for example, an intravascular ultrasound imaging system which forms an image by using ultrasound signals.

As illustrated in Fig. 1, the optical coherence tomography apparatus 500 includes a catheter unit 100 attachably and detachably serving as an optical probe, a scanner & pull-back unit 1, and an operation control apparatus 501. The scanner & pull-back unit 1 and the operation control apparatus 501 are connected to each other through a signal cable 502.

The catheter unit 100 is directly inserted into a blood vessel of a patient, and the state inside the blood vessel is measured by using low coherent light emitted from an optical imaging core. A connection section 201 of the catheter unit 100 is attachably and detachably connected to a catheter connection section 7 of the scanner & pull-back unit 1. The scanner & pull-back unit 1 is also referred to as a motor drive unit (MDU) or a drive device and executes radial scanning of the optical imaging core inside the catheter unit 100.

The operation control apparatus 501 illustrated in Fig. 1 has a function of inputting various types of setting values during optical coherence tomographic diagnosis, a function of processing data obtained through measurement and displaying the data as a cross-sectional image, and the like. The operation control apparatus 501 has a main body control unit 503, an LCD monitor 504, an operation panel 505, and printer and recorder 506. The main body control unit 503 performs processing of the data obtained through measurement and outputs a processing result. The LCD monitor 504 displays the processing result. A user inputs various types of the setting values through the operation panel 505. The printer and recorder 506 print the processing result and store the processing result as data.

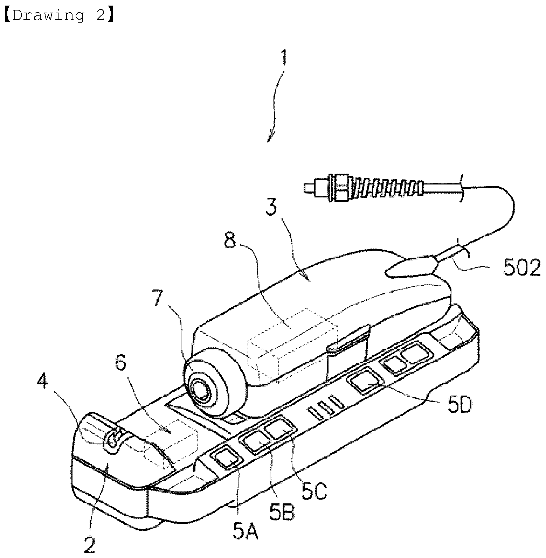

Next, with reference to Fig. 2, an example of the scanner & pull-back unit 1 will be described. Fig. 2 is a perspective view illustrating the appearance of an example of the scanner & pull-back unit 1 illustrated in Fig. 1.

The scanner & pull-back unit 1 illustrated in Fig. 2 has a pull-back unit 2 and a scanner unit 3. The pull-back unit 2 has a catheter clamp 4, operation buttons 5A to 5D, a pull-back motor 6, and the like. The scanner unit 3 has the catheter connection section 7 and a scanner motor 8.

The scanner & pull-back unit 1 illustrated in Fig. 2 emits near infrared light output by a wavelength variable semiconductor laser therein, with respect to a vascular wall through the catheter unit 100 illustrated in Fig. 1. The scanner & pull-back unit 1 changes reflected light received from a target into a signal and sends the signal to the main body control unit 503 through the signal cable 502. The main body control unit 503 acquires an optical coherence tomographic image (OCT image) by performing processing of the obtained signal. For example, the scanner & pull-back unit 1 divides the near infrared light output by the wavelength variable semiconductor laser into two routes for measurement light and reference light. The measurement light is radially emitted to a vascular wall through a designated catheter unit 100 in a rotating manner, and reflected light thereof is acquired through the catheter unit 100. The main body control unit 503 performs Fourier transformation of a coherence signal generated by causing the acquired reflected light and reference light to interfere with each other. The main body control unit 503 obtains reflectance information regarding the depth of the vascular wall and arranges the information in the rotary direction, thereby obtaining an optical coherence tomographic image of the blood vessel.

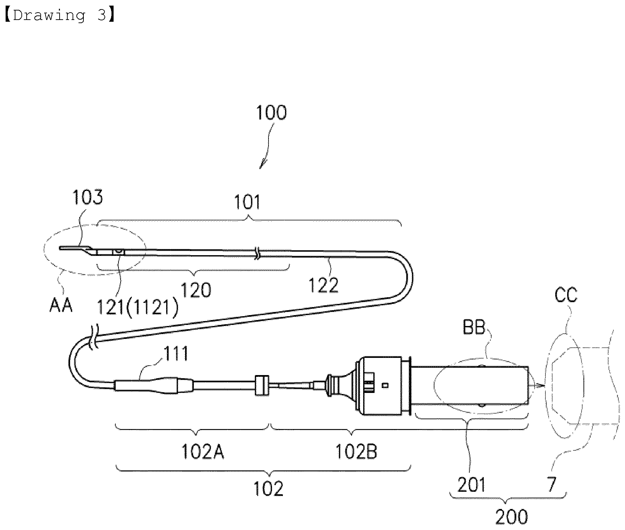

Fig. 3 is a view illustrating a structural example of the catheter unit 100 illustrated in Fig. 1.

The catheter unit 100 illustrated in Fig. 3 has a catheter sheath 101 and a connector portion 102. A distal portion of the catheter sheath 101 is provided with a tube 103 configuring a guide wire lumen. The catheter sheath 101 is continuously formed from the tube 103 to the connector portion 102.

A light transmitting and receiving unit 121 transmitting and receiving measurement light, and an optical fiber cable are accommodated inside the catheter sheath 101 illustrated in Fig. 3. An optical imaging core 120 is disposed substantially throughout the overall length of the catheter sheath 101. The optical imaging core 120 includes a coiled drive shaft 122 for transmitting a drive force which rotates the optical fiber cable.

The connector portion 102 illustrated in Fig. 3 includes a sheath connector 102A and a drive shaft connector 102B. The drive shaft connector 102B is fixed to a proximal portion of the drive shaft 122.

A distal portion of the drive shaft connector 102B is attachably and detachably connected to a catheter connection holding body 7 of the scanner & pull-back unit 1 illustrated in Fig. 2. In a connection device 200 of the embodiment of the present invention, when the connection section 201 of the drive shaft connector 102B and the catheter connection holding body 7 of the scanner & pull-back unit 1 are connected to each other, an end portion of the optical fiber cable inside the connection section 201 and the end portion of the optical fiber cable inside the catheter connection holding body 7 can be connected and coupled to each other. The optical fiber cables can be reliably and smoothly connected to each other while jamming is prevented when the connection section 201 and the catheter connection holding body 7 are coupled to each other.

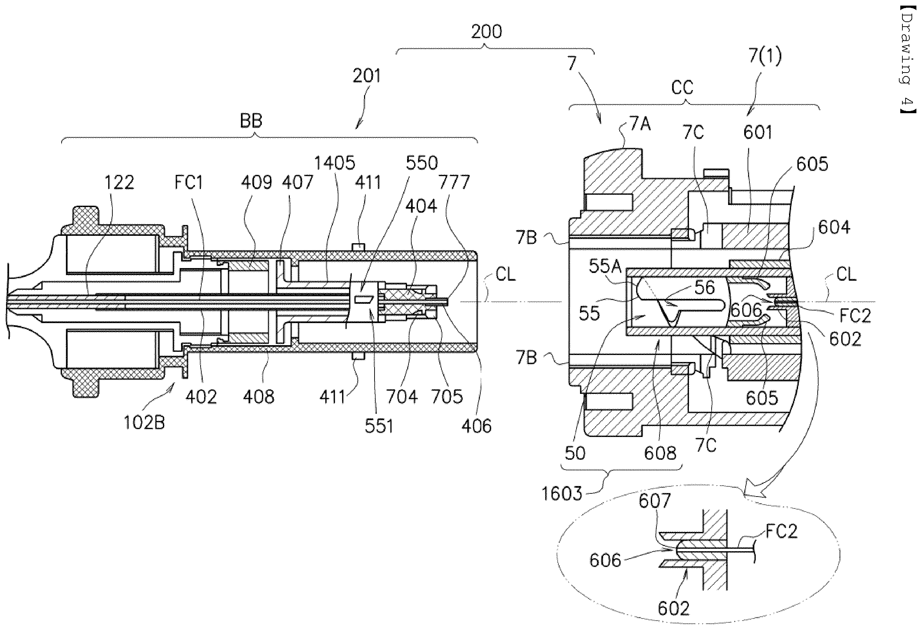

Fig. 4 is a cross-sectional view illustrating a structural example of a regional portion BB of the connection section 201 of the drive shaft connector 102B and a regional portion CC of the catheter connection holding body 7 of the scanner & pull-back unit 1 illustrated in Fig. 3.

The connection section 201 of the drive shaft connector 102B and the catheter connection holding body 7 of the scanner & pull-back unit 1 illustrated in Fig. 4 configure the connection device 200 of the embodiment of the present invention. In the connection device 200, an end portion 777 of an optical fiber cable FC1 positioned on a central axis CL of the connection section 201 of the drive shaft connector 102B, and an end portion 607 of an optical fiber cable FC2 positioned on the central axis CL of the catheter connection holding body 7 of the scanner & pull-back unit 1 can be connected to each other by being brought into contact with each other.

The connection section 201 of the drive shaft connector 102B is also referred to as a connector, and the catheter connection holding body 7 of the scanner & pull-back unit 1 can be referred to as an adapter for receiving the connection section 201 which is the connector. For example, the optical fiber cables FC1 and FC2 on one side and the other side are single mode optical fiber cables and each of which is configured to include a core for transmitting light, and a clad having a refractive index slightly smaller than that of the core. A resin coating material is provided on the outer peripheral surface of the clad.

First, the structural example of the connection section 201 of the drive shaft connector 102B illustrated in Fig. 4 will be described. A connector member 404 for the optical fiber cable FC1 is joined to the drive shaft 122 via a connection pipe 402. A connector fixing member 1405 is a cylindrical member and has a disk-shaped flange 407 at an end portion thereof. The flange 407 is formed at the end portion of the connector fixing member 1405. The connector member 404 is fixed to the inside of the cylindrical connector fixing member 1405. The connector fixing member 1405 is rotatably held inside a housing 408 of the drive shaft connector 102B. A pair of projection portions 411 are respectively formed at positions opposite to each other on the outer peripheral surface of the housing 408.

As illustrated in Fig. 4, a ferrule 406 is provided at the distal end of the connector member 404, and the ferrule 406 is fixed to the end portion of the optical fiber cable FC1. A protruding portion 704 and a round projection 705 are formed at the distal portion of the connector member 404. In order to prevent noise from being generated due to reflection of light on the end surface, the end portion 777 of the optical fiber cable FC1 is subjected to APC-type processing in which a slope angle is formed with respect to a traveling direction of light.

As illustrated in Fig. 4, inside the housing 408, an elastic member 409 is disposed at a position in the vicinity of the flange 407 so as to be able to be in contact therewith. The elastic member 409 pressurizes the flange 407 when the connection section 201 of the drive shaft connector 102B and the catheter connection holding body 7 of the scanner & pull-back unit 1 are connected to each other, thereby facilitating the operation of connecting the end portion 777 of the optical fiber cable FC1 and the end portion 607 of the optical fiber cable FC2 to each other. After the end portion 777 of the optical fiber cable FC1 and the end portion 607 of the optical fiber cable FC2 are connected (coupled) to each other, the elastic member 409 and the flange 407 are in a non-contact state. Accordingly, the inner members can be prevented from being damaged or deformed during the internal driving. For example, the elastic member 409 is synthetic rubber, a metal spring, silicon rubber having low viscosity, or the like.

Next, the structural example of the catheter connection holding body 7 of the scanner & pull-back unit 1 illustrated in Fig. 4 will be described.

A housing 601 illustrated in Fig. 4 is fixed to the inside of a head portion 7A of the catheter connection holding body 7. The housing 408 of the drive shaft connector 102B is fitted to the inner surface of the housing 601 when the connectors are connected to each other. The head portion 7A internally has a groove portion entrance 7B. The housing 601 is provided with a pair of groove portions 7C leading from the groove portion entrance 7B of the head portion 7A. The pair of groove portions 7C can receive the pair of projection portions 411 of the housing 408.

An adapter member 602 illustrated in Fig. 4 is a portion to be coupled to the connector member 404 and is held so as to be relatively rotatable with respect to the housing 601. An adapter fixing member 1603 is a cylindrical member and the adapter member 602 is fixed to the inside thereof so as not to be relatively rotatable. That is, the adapter member 602 and the adapter fixing member 1603 are integrated with each other. The adapter fixing member 1603 performs positioning of the connector member 404 in the circumferential direction in association with the connector fixing member 1405 while being coupled to the connector member 404.

The adapter fixing member 1603 is joined to a drive force relay pipe 604. A drive force of the rotary driving scanner motor 8 illustrated in Fig. 2 is sent to the adapter fixing member 1603 via the drive force relay pipe 604 and is transmitted to the drive shaft 122 after being coupled to the connector member 404. As the adapter fixing member 1603, a 0 dB-attenuator (0 dB-ATT) is employed. However, the adapter fixing member 1603 is not limited thereto.

As illustrated in Fig. 4, a pair of claw portions 605 are formed on the inner surface of the adapter fixing member 1603. The pair of claw portions 605 engage with the connector member 404, thereby causing the connector member 404 and the adapter member 602 to be rigidly integrated with each other in an attachable and detachable manner. A female-type hole 606 receiving the ferrule 406 of the connector member 404 is formed in the adapter member 602, and the end portion 607 of the optical fiber cable FC2 subjected to APC-type processing is fixed to the inner side of the hole 606.

The adapter fixing member 1603 illustrated in Fig. 4 has an outer tubular protective tube 608 and an inner tubular guide member 50. The guide member 50 is fixed to the inner surface of the protective tube 608 or is formed so as to be integrated therewith.

When the connection section 201 of the drive shaft connector 102B and the catheter connection holding body 7 of the scanner & pull-back unit 1 are joined to each other, the guide member 50 guides the connector fixing member 1405 while relatively gyrating around the central axis CL. Accordingly, the guide member 50 plays a role of positioning around the central axis CL in a gyratory direction by reliably and smoothly guiding the connector fixing member 1405 into the adapter fixing member 1603.

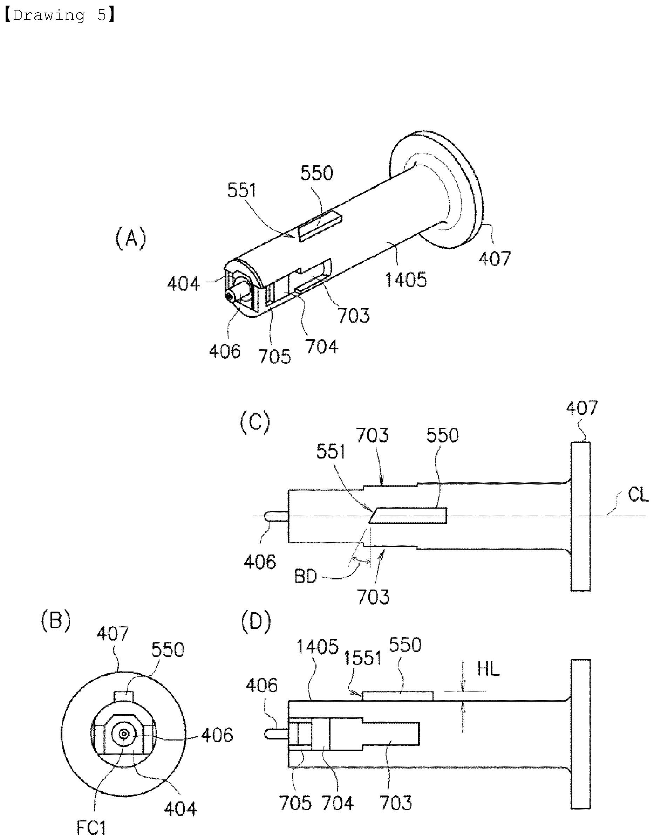

Next, with reference to Fig. 5, a shape of the connector fixing member 1405 illustrated in Fig. 4 will be further described. Fig. 5 illustrates a shape of the connector fixing member 1405.

As described above, the connector fixing member 1405 has the flange 407, symmetrical slits 703 and 703 on the right and left, and one projection portion 550. The connector member 404 is disposed inside the connector fixing member 1405, and the ferrule 406 protrudes from the connector member 404. The outer peripheral surface of the connector fixing member 1405 is provided with the projection member (also referred to as the guide key) 550.

The projection portion 550 is formed so as to be parallel to the central axis CL of the connector fixing member 1405, and the front end portion of the projection portion 550 forms one slope portion 551. As illustrated in Fig. 5(C), the one slope portion 551 is formed so as to have a slope angle BD (acute angle) with respect to a line orthogonal to the central axis CL. The projection portion 550 is formed at a position separated from each of the slits 703 and 703 on the right and left by 90 degrees of angle around the central axis CL in the circumferential direction.

Moreover, as illustrated in Fig. 5(D), a forming height HL of the projection portion 550 is highly set in order to prevent an occurrence of a state where the projection portion 550 is drawn into the inner surface side of the guide member 50 and causes jamming therein. For example, the forming height HL is set so as to be the same as or greater than the thickness of the guide member 50.

In each of the slits 703, the outer peripheral surface of the connector member 404 is partially exposed. The protruding portion 704 and the round projection 705 are formed on the outer peripheral surface of the connector member 404. The protruding portion 704 and the round projection 705 are positioned inside the slit 703.

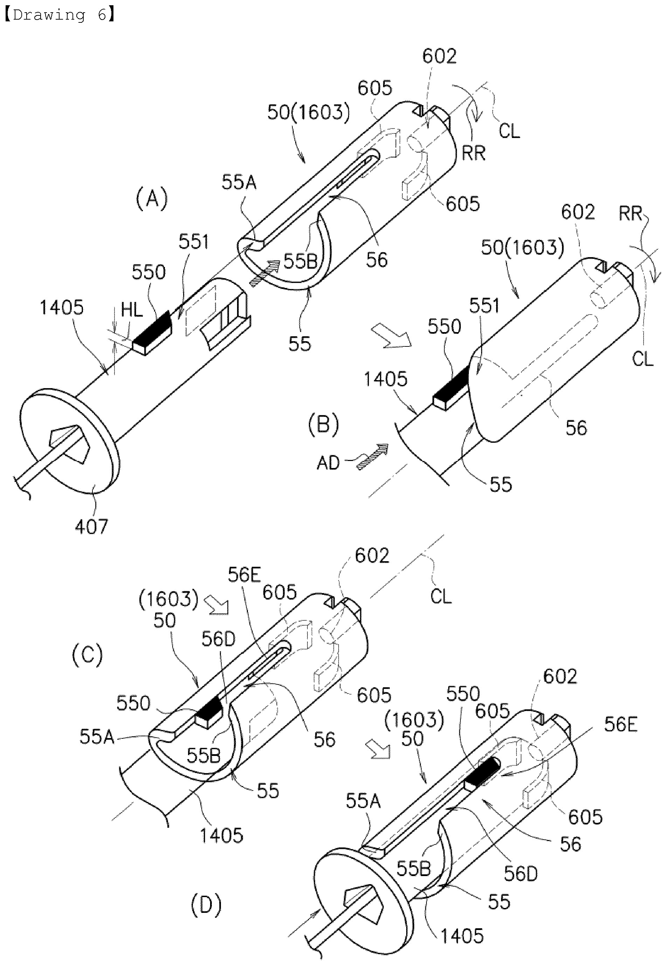

Next, with reference to Fig. 6, an example of a shape of the guide member 50 of the adapter fixing member 1603 illustrated in Fig. 4 according to the invention will be described. Fig. 6 is a perspective view illustrating the connector fixing member 1405 illustrated in Fig. 4, and the guide member 50 of the adapter fixing member 1603. In Fig. 6, in order to make the view easy to understand, illustration of the protective tube 608 illustrated in Fig. 4 is omitted, and only the guide member 50 of the adapter fixing member 1603 is illustrated.

In Figs. 6(A) and 6(B), it is preferable that the guide member 50 of the adapter fixing member 1603 is rotated on the guide member 50 side of the adapter fixing member 1603 in accordance with driving of the motor (the scanner motor 8 illustrated in Fig. 2) at a low speed, and the guide member 50 of the adapter fixing member 1603 and the connector fixing member 1405 are relatively rotated in a gyratory direction RR by an angle approximately a little less than 360 degrees. Accordingly, as illustrated in Figs. 6(C) and 6(D), the guide member 50 of the adapter fixing member 1603 and the connector fixing member 1405 are positioned with respect to the gyratory direction RR and the central axis CL, thereby being connected to each other.

First, as illustrated in Fig. 6(A), the guide member 50 is a cylindrical member. The guide member 50 has a helical guide following surface 55 and a guide groove portion 56. The helical guide following surface 55 is formed so as to slope along one direction around the central axis CL from a helical vertex portion 55A to a helical rear end portion 55B and is smoothly formed at a predetermined slope angle, for example, a slope angle of 15 degrees. The guide groove portion 56 is formed so as to be parallel to the central axis CL. The groove width of the guide groove portion 56 is formed by a line extending in a direction of the central axis CL from the helical vertex portion 55A, and a line extending from the helical rear end portion 55B. That is, the helical vertex portion 55A and the guide groove portion 56 are adjacent to each other, and the low point of the helix and the guide groove portion 56 are also adjacent to each other. In other words, the guide groove portion 56 is positioned between the helical vertex portion 55A and the low point of the helix.

The slope portion 551 of the projection portion 550 is formed in accordance with the slope angle of the helical guide following surface 55. Accordingly, the slope portion 551 of the projection portion 550 is smoothly guided along the helical guide following surface 55 from the helical vertex portion 55A to the helical rear end portion 55B. Moreover, the projection portion 550 can be introduced into the guide groove portion 56.

In addition, as illustrated in Fig. 4, in consideration of the safety, the protective tube 608 covers the guide member 50 such that even though the helical vertex portion 55A of the guide following surface 55 is sharp, a user's finger by no means touches the helical vertex portion 55A. Moreover, since the protective tube 608 is provided, the helical vertex portion 55A is prevented from hitting a certain object and being damaged. In addition, the protective tube 608 functions to guide the connector fixing member 1405 when the connector fixing member 1405 is inserted along the central axis CL.

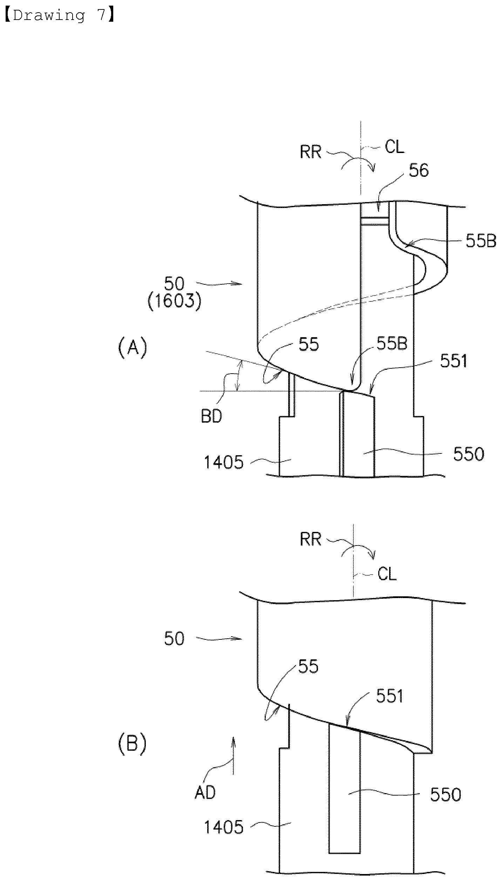

Next, with reference to Figs. 6 and 7, description will be given regarding an example of a connection operation performed along the gyratory direction RR in which the connector fixing member 1405 and the guide member 50 of the adapter fixing member 1603 are coupled to each other. Figs. 7 (A) and 7 (B) illustrate a state where the guide member 50 of the adapter fixing member 1603 rotates. In this case, it is preferable that the guide member 50 is rotated by driving the motor (the scanner motor 8 illustrated in Fig. 2) at a low speed.

When a user holds the connection section 201 of the drive shaft connector 102B illustrated in Fig. 3, by hand and performs connection by causing the connection section 201 thereof to be fitted to the catheter connection holding body 7 of the scanner & pull-back unit 1, as illustrated in Fig. 6(A), the distal portion of the connector fixing member 1405 approaches the guide member 50 of the adapter fixing member 1603. Accordingly, as illustrated in Fig. 7(A), the slope portion 551 of the projection portion 550 of the connector fixing member 1405 bumps into the helical vertex portion 55A of the guide following surface 55.

In response to driving of the scanner motor 8 illustrated in Fig. 2, the guide member 50 of the adapter fixing member 1603 rotates around the central axis CL in the gyratory RR direction at a low speed. Therefore, as illustrated in Figs. 6 (B) and 7(B), the slope portion 551 of the projection portion 550 is guided along the helical rear end portion 55B from the helical vertex portion 55A of the guide following surface 55. Accordingly, the connector fixing member 1405 advances in an insertion direction AD.

Then, gyrating of the guide member 50 of the adapter fixing member 1603 in the gyratory direction RR proceeds, and as illustrated in Fig. 6(C), the slope portion 551 of the projection portion 550 arrives at a position of a distal portion 56D of the guide groove portion 56 from the helical rear end portion 55B. Here, the user further presses the connection section 201 of the drive shaft connector 102B in Fig. 3 by hand, and as illustrated in Fig. 6(D), the projection portion 550 is guided to an inner end portion 56E of the guide groove portion 56 and bumps into the inner end portion 56E.

Accordingly, the pair of claw portions 605 of the adapter fixing member 1603 illustrated in Fig. 4 individually engage with the protruding portion 704 of the connector member 404, and the ferrule 406 is fitted to the adapter member 602. The end portion of the optical fiber cable FC1 and the end portion of the optical fiber cable FC2 can be reliably and smoothly coupled to each other.

In this manner, when the user holds the connection section 201 of the drive shaft connector 102B illustrated in Fig. 3, by hand and mounts the connection section 201 thereof in the catheter connection holding body 7 of the scanner & pull-back unit 1, in regard to the below-described point, the end portion 777 of the optical fiber cable FC1 and the end portion 607 of the optical fiber cable FC2 illustrated in Fig. 4 can be reliably and smoothly connected to each other.

As illustrated in Figs. 6 and 7, one helical guide following surface 55 is sloped along only one direction and is smoothly formed around the central axis CL from the helical vertex portion 55A to the helical rear end portion 55B in a continuously helical manner. The slope portion 551 of the projection portion 550 is formed in accordance with the slope angle BD of the helical guide following surface 55. Accordingly, the slope portion 551 of the projection portion 550 is smoothly guided along the helical guide following surface 55 from the helical vertex portion 55A to the helical rear end portion 55B, and thus, the slope portion 551 can be introduced into the guide groove portion 56.

In addition, as illustrated in Fig. 6, the forming height HL of the projection portion 550 is highly set in order to prevent an occurrence of a state where the projection portion 550 is drawn into the inner surface side of the guide member 50 and is jammed therein. Accordingly, the projection portion 550 can avoid a state of being drawn into the inner surface of the guide member 50 and causing jamming therein. If the projection member is drawn into the inner surface of the guide member and causes jamming therein, there is concern that the guide member, the protective member, and peripheral portions thereof may be damaged. When damage occurs, there is a need to perform a repair, and the scanner & pull-back unit 1 illustrated in Fig. 1 cannot be used any longer.

Figs. 8 to 10 illustrate a structural example of connecting and fixing the connection section 201 of the drive shaft connector 102B and the catheter connection holding body 7 of the scanner & pull-back unit 1 to each other. Here, with reference to Figs. 8 to 10, description will be given regarding an example of a procedure of connecting and fixing the connection section 201 of the drive shaft connector 102B and the catheter connection holding body 7 of the scanner & pull-back unit 1 to each other. The structure of connecting and fixing thereof is not limited to the illustrated example, and an arbitrary structure can be employed.

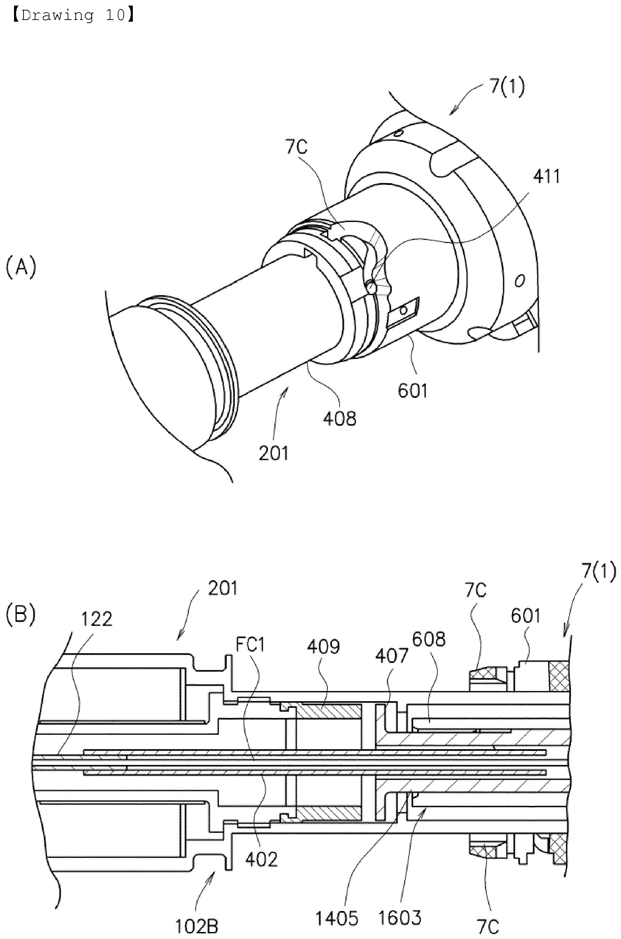

Fig. 8 is a view illustrating a state immediately before the connection section 201 of the drive shaft connector 102B and the catheter connection holding body 7 of the scanner & pull-back unit 1 are connected and fixed to each other. Fig. 9 is a view illustrating a state in the middle of an operation of fixing the connection section 201 of the drive shaft connector 102B and the catheter connection holding body 7 of the scanner & pull-back unit 1 to each other. Fig. 10 is a view illustrating a state where the connection section 201 of the drive shaft connector 102B and the catheter connection holding body 7 of the scanner & pull-back unit 1 are fixed to each other.

As illustrated in Figs. 8(A) and 8(B), the pair of projection portions 411 of the connection section 201 are inserted along an insertion direction T with respect to the groove portions 7C of the catheter connection holding body 7. At this time, as illustrated in Fig. 8(B), the flange 407 and the elastic member 409 are in a non-contact state.

Next, as illustrated in Figs. 9(A) and 9(B), when the housing 408 of the connection section 201 is further inserted into the insertion direction T and the projection portions 411 are respectively positioned at the deepest positions in the groove portions 7C, as illustrated in Fig. 9(B), the flange 407 pressurizes the elastic member 409 and the elastic member 409 is compressed. At this time, the end portion 777 of the optical fiber cable FC1 and the end portion 607 of the optical fiber cable FC2 illustrated in Fig. 4 are connected to each other as described above.

Moreover, as illustrated in Figs. 10(A) and 10 (B), when the projection portions 411 are caused to slide along the groove portions 7C, the projection portions 411 advance in the gyratory direction perpendicular to the insertion direction T, and the projection portions 411 stop at a trailing end portion of the groove portion 7C. At this time, as illustrated in Fig. 10 (B), the flange 407 and the elastic member 409 are separated from each other and return to the non-contact state.

In this manner, the connection section 201 of the drive shaft connector 102B and the connection holding body 7 of the scanner & pull-back unit 1 can be connected and fixed to each other.

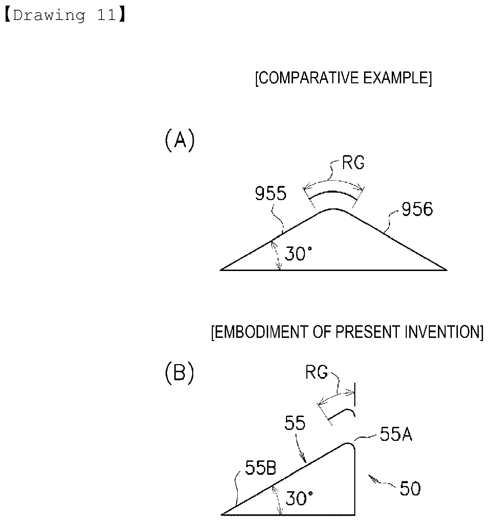

Here, with reference to Fig. 11, the first embodiment of the present invention and a comparative example which is beyond the scope of the present invention will be described.

In the comparative example illustrated in Fig. 11(A), two sloped guide following surfaces 955 and 956 are formed. In the guide following surfaces 955 and 956, a range RG in which a connector fixing member may cause jamming is wide.

In contrast, in the embodiment of the present invention illustrated in Fig. 11(B), in the guide member 50, since there is one sloped guide following surface 55 formed in only one direction, a range RT in the guide following surface 55 can be narrower than the range RG.

As described above, one sloped guide following surface 55 is employed such that the connector fixing member 1405 can rotate along a direction rotating around the central axis CL. Moreover, the forming height HL of the connector fixing member 1405 is set to be significant as much as possible. Accordingly, the connector fixing member 1405 can be reliably prevented from being drawn into the inner surface side of the guide member 50 and causing jamming therein.

Figs. 12(A) and 12(B) respectively illustrate cases where the slope angle of one guide following surface 55 of the guide member 50 is 30 degrees and 15 degrees. A force F1 which is illustrated in Fig. 12(A), is generated in a case where the slope angle is 30 degrees, and thrusts the catheter (the connector fixing member 1405) can be smaller than a force F2 which is illustrated in Fig. 12(B), is generated in a case where the slope angle is 15 degrees, and thrusts the catheter (the connector fixing member 1405).

That is, as the slope angle of the one guide following surface 55 increases, a force that can guide the catheter (the connector fixing member 1405) can be generated by a smaller force of thrusting the catheter (the connector fixing member 1405), and thus, as seen from the comparison between a frictional force MF1 and a frictional force MF2, a generated frictional force can also be reduced. Therefore, when the slope angle is significant, connecting work of connecting the end portion 777 of the optical fiber cable FC1 and the end portion 607 of the optical fiber cable FC2 illustrated in Fig. 4 connecting work can be more easily performed.

### (Second Embodiment)

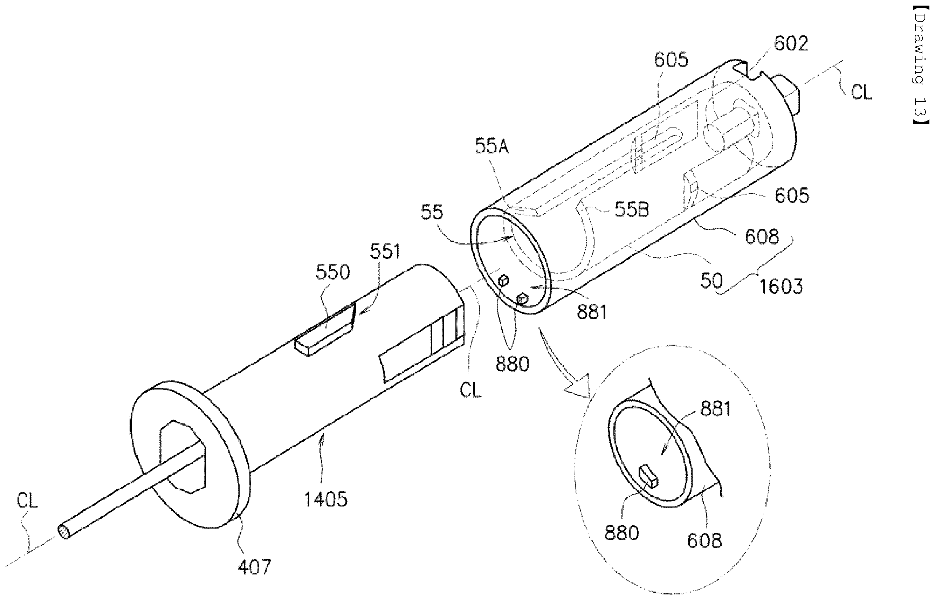

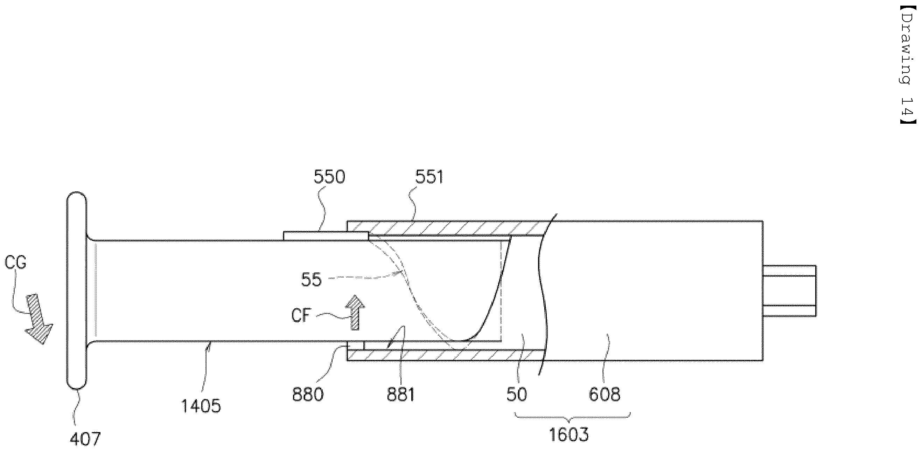

Next, with reference to Figs. 13 and 14, a second embodiment of the present invention will be described.

Fig. 13 is a perspective view illustrating the connector fixing member 1405 of the second embodiment of the present invention, and the adapter fixing member 1603. Fig. 14 is a side view having a cross section illustrating a state where the connector fixing member 1405 is inserted into the adapter fixing member 1603.

The structures of the connector fixing member 1405 of the second embodiment of the present invention illustrated in Figs. 13 and 14, and the guide member 50 of the adapter fixing member 1603 are the same as those of the connector fixing member 1405 illustrated in Fig. 7, and the guide member 50 of the adapter fixing member 1603. The adapter fixing member 1603 has the outer tubular protective tube 608 and the inner tubular guide member 50. The guide member 50 is fixed to the inner surface of the protective tube 608 or is formed so as to be integrated therewith.

However, in the second embodiment of the present invention, one or a plurality of slope restraining projection portions 880 are additionally provided on the inner surface of the protective tube 608 of the adapter fixing member 1603. The slope restraining projection portion 880 is formed so as to protrude on an inner surface 881 of an opening distal portion of the protective tube 608. The position where the projection portion 880 is formed is a position facing the helical vertex portion 55A of the one guide following surface 55, and is a position where the guiding projection portion 550 does not hit the slope restraining projection portion 880 when the connector fixing member 1405 is pulled out from the inside of the protective tube 608. Accordingly, when the connector fixing member 1405 is detached, the connector fixing member 1405 can be smoothly pulled out so as not to hit the projection portion 880 from the inside of the protective tube 608.

As illustrated in Figs. 13 and 14, on the inner surface of the protective tube 608 of the adapter fixing member 1603, one or the plurality of slope restraining projection portions 880 are provided. As illustrated in Fig. 14, even though a force CG that tends to cause the flange 407 side of the connector fixing member 1405 to slope tends to be generated when the connector fixing member 1405 is intended to be inserted in the inner surface of the protective tube 608 of the adapter fixing member 1603, the projection portion 880 causes a force CF that restrains sloping to act on the connector fixing member 1405.

Therefore, the projection portion 880 restrains the force CG that tends to cause the flange 407 side of the connector fixing member 1405 to slope from being generated, and thus, it is possible to further prevent a phenomenon in which the guiding projection portion 550 of the connector fixing member 1405 is drawn into the inner surface of the guide member 50 and causes jamming therein.

### (Third Embodiment)

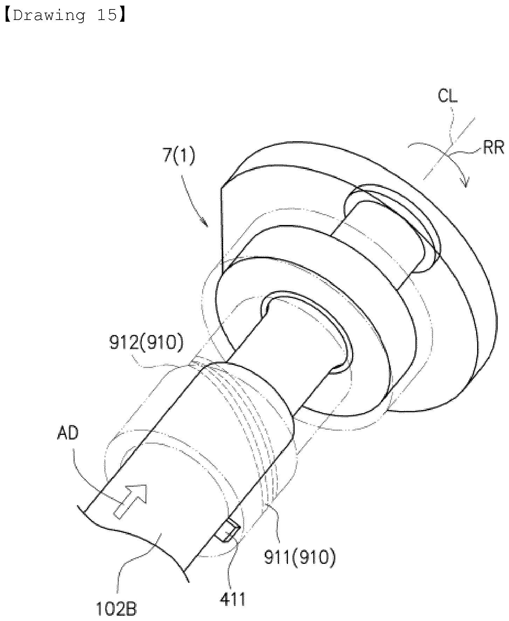

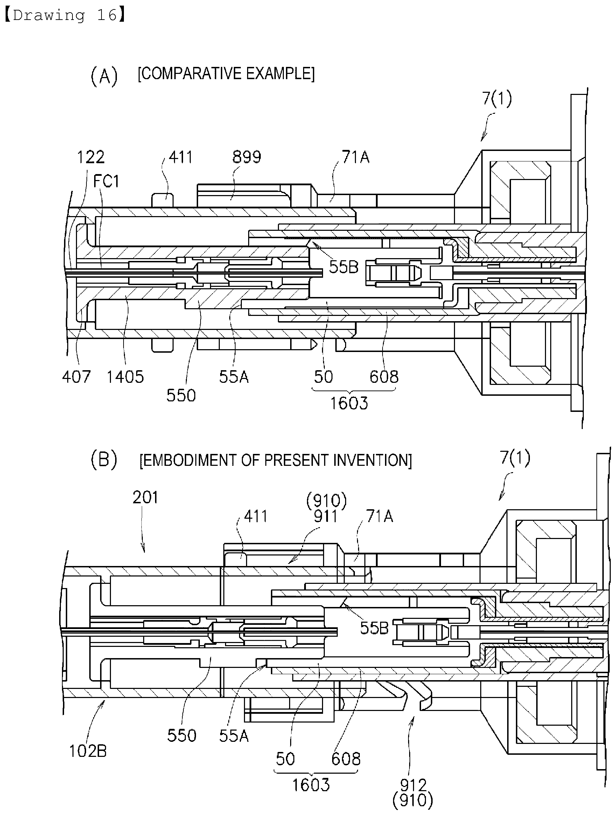

Next, with reference to Figs. 15 and 16, a third embodiment of the present invention will be described.

Figs. 15 and 16(B) illustrate a structural example in which the connection section 201 of the drive shaft connector 102B of the third embodiment of the present invention, and the catheter connection holding body 7 of the scanner & pull-back unit 1 are connected and fixed to each other. Fig. 15(A) illustrates a different structural example for comparison.

As illustrated in Figs. 15 and 16(B), a helical guide groove portion 910 is formed in the catheter connection holding body 7. The helical guide groove portion 910 is formed around the central axis CL in a helical manner and is formed in the same gyratory direction as that of the helical guide following surface 55 of the guide member 50. The helical guide groove portion 910 has a leading end portion 911 lead to a trailing end portion 912.

Accordingly, as illustrated in Fig. 16 (B), at the point of time the projection portions 411 of the connection section 201 of the drive shaft connector 102B enters the leading end portion 911 of the helical guide groove portion 910, the guiding projection portion 550 of the connector fixing member 1405 has not yet bumped into the helical vertex portion 55A of the helical guide following surface 55 of the guide member 50, and a gyration guiding operation for the projection portions 411 of the connection section 201 of the drive shaft connector 102B is performed prior thereto along the helical guide groove portion 910 from the leading end portion 911 of the helical guide groove portion 910 and proceeds along the central axis CL. That is, the projection portions 411 of the connection section 201 take the lead and start a gyration guiding operation.

When the guiding projection portion 550 illustrated in Fig. 16 (B) hits a middle portion of the helical guide following surface 55 of the guide member 50 (a position avoiding the helical vertex portion 55A), the guiding projection portion 550 is guided by the helical guide following surface 55 and starts a gyration guiding operation around the central axis CL.

Accordingly, after the projection portion 411 of the connection section 201 take the lead and start the gyration guiding operation, the guiding projection portion 550 can be smoothly guided in a manner of gyrating from the middle portion of the helical guide following surface 55 to the helical rear end portion 55B. Therefore, end portions 777 and 607 of the optical fiber cables FC1 and FC2 can be reliably and smoothly connected to each other.

In contrast, in the comparative example illustrated in Fig. 16(A), the guiding projection portion 550 bumps into the helical vertex portion 55A of the helical guide following surface 55 of the guide member 50 in advance, and the projection portions 411 of the connection section 201 have not yet entered a helical guide groove portion 899.

### (Fourth Embodiment)

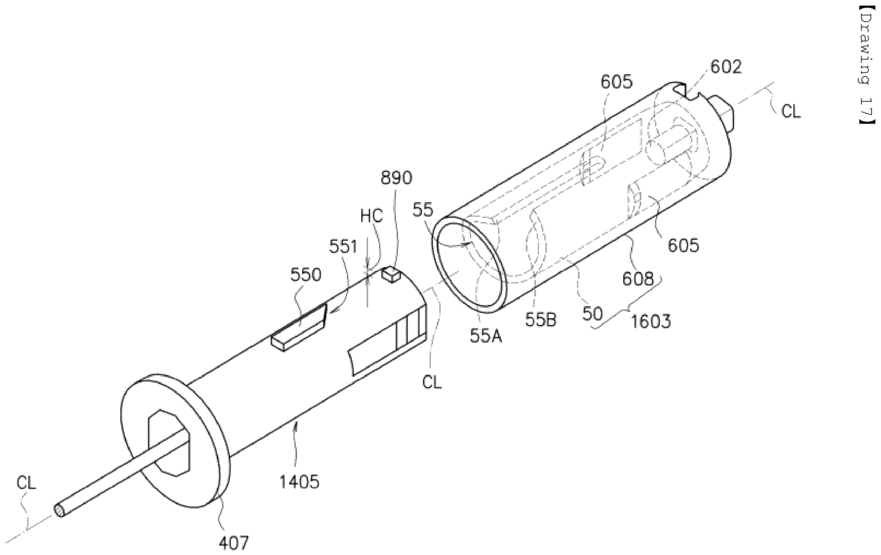

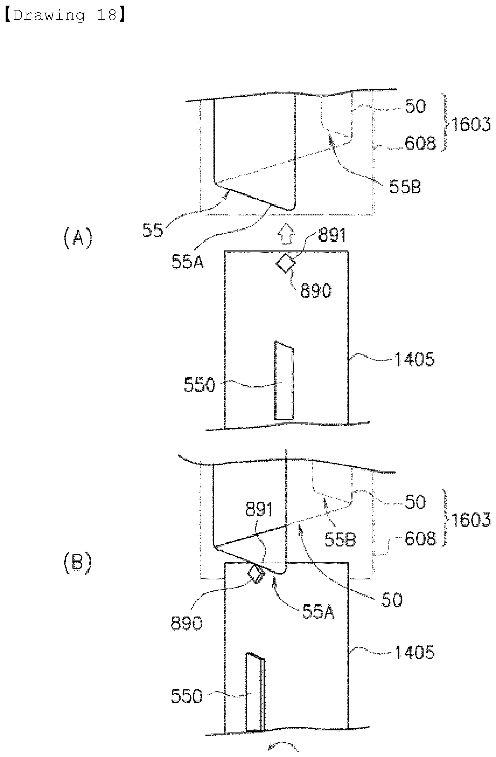

Next, with reference to Figs. 17 and 18, a fourth embodiment of the present invention will be described.

Fig. 17 is a perspective view illustrating the connector fixing member 1405 of the fourth embodiment of the present invention, and the adapter fixing member 1603. Fig. 18 is a view illustrating a state where the connector fixing member 1405 is inserted into the adapter fixing member 1603.

The structures of the connector fixing member 1405 illustrated in Figs. 17 and 18, and the guide member 50 of the adapter fixing member 1603 are the same as those of the connector fixing member 1405 illustrated in Fig. 7, and the guide member 50 of the adapter fixing member 1603. The adapter fixing member 1603 has the outer tubular protective tube 608 and the inner tubular guide member 50. The guide member 50 is fixed to the inner surface of the protective tube 608 or is formed so as to be integrated therewith.

However, in the fourth embodiment of the present invention, a leading guide projection 890 is additionally formed at a position of the distal portion on the outer peripheral surface of the connector fixing member 1405. The guide projection 890 is formed at a position ahead of the guiding projection portion 550 of the connector fixing member 1405 (distal portion side) along the central axis CL, and the guide projection 890 is positioned in the distal portion so as to be able to take the lead prior to the guiding projection portion 550.

For example, the guide projection 890 is formed so as to have a square shape as illustrated in Fig. 18. However, the shape is not particularly limited. A distal surface 891 of the guide projection 890 is sloped with respect to the central axis CL. A protrusion forming height HC of the guide projection 890 may be the same as or slightly smaller than the thickness of the guide member 50.

Accordingly, when the connector fixing member 1405 is inserted into the tubular protective tube 608 of the adapter fixing member 1603, the guide projection 890 takes the lead prior to the guiding projection portion 550 and hits the helical vertex portion 55A of the helical guide following surface 55. Therefore, it is possible to induce rotation along the helical guide following surface 55 of the guide member 50. That is, before the guiding projection portion 550 hits the helical vertex portion 55A, the guide projection 890 bumps into the helical vertex portion 55A. Therefore, the guiding projection portion 550 can avoid directly hitting the helical vertex portion 55A. Accordingly, the guiding projection portion 550 can be caused to smoothly copy the helical guide following surface 55 from the position where the guiding projection portion 550 has avoided the helical vertex portion 55A of the helical guide following surface 55.

Incidentally, in the above-described embodiments, for example, as exemplified in Figs. 4 and 6, one guide groove portion (also referred to as the slot) 56 is provided in the guide member 50. In each of the embodiments, without being limited thereto, two or more, that is, a plurality of the guide groove portions 56 may be provided in the guide member 50.

The connection device 200 according to the embodiment of the present invention is a connection device which connects an end portion of a first optical fiber cable FC1 as a first information transmission cable and an end portion of a second optical fiber cable FC2 as a second information transmission cable to each other. The connection device 200 includes the connection section 201 that holds the tubular connector fixing member 1405 holding the first optical fiber cable FC1 and having the projection portion 550 on the outer peripheral surface, and the tubular adapter fixing member 1603 that holds the second optical fiber cable FC2 and connects the end portion of the first optical fiber cable FC1 and the end portion of the second optical fiber cable FC2 to each other in accordance with insertion of the connector fixing member 1405. The adapter fixing member 1603 internally has the guide member 50. The guide member 50 is provided with the helical guide following surface 55 formed in one direction in order to perform an operation in which the projection portion 550 of the connector fixing member 1405 is guided in a manner of gyrating around the central axis CL of the first optical fiber cable FC1 and the second optical fiber cable FC2 when the connector fixing member 1405 is inserted.

Accordingly, when the connector fixing member 1405 is inserted into the guide member 50 in order to connect the end portion of the first optical fiber cable FC1 and the end portion of the second optical fiber cable FC2 to each other, the projection portion 550 of the connector fixing member 1405 can be guided in a manner of gyrating along the helical guide following surface 55 formed in one direction in the guide member 50 without confusing the gyratory direction.

That is, the projection portion 550 of the connector fixing member 1405 can be reliably guided in a manner of gyrating in only one direction along the helical guide following surface 55 formed in one direction in the guide member 50. Accordingly, in the present invention, the connector fixing member 1405 and the guide member 50 can be smoothly coupled to each other, and the optical fiber cables can be reliably connected to each other while jamming is prevented from occurring in the connection device.

In contrast, in a case of a structure in the related art, a connector fixing member 405 gyrates in any direction of slope end surfaces 801A and 801B respectively in two directions. Since there are two slope surfaces, the angle of a sharp distal portion is significant, and there is concern that jamming may occur in the connection device.

As illustrated in Figs. 13 and 14, the inner surface of the adapter fixing member 1603 is provided with the slope restraining projection portion 880 which restrains the connector fixing member 1405 from sloping with respect to the adapter fixing member 1603 when the connector fixing member 1405 is inserted into the guide member 50.

Accordingly, even though a force that tends to cause the connector fixing member 1405 to slope tends to be generated when the connector fixing member 1405 is intended to be inserted in the inner surface of the adapter fixing member 1603, the projection portion 880 causes a force that restrains sloping to act on the connector fixing member 1405. Therefore, the projection portion 880 restrains a force that tends to cause the connector fixing member 1405 to slope from being generated, and thus, it is possible to further prevent a phenomenon in which the projection portion 550 of the connector fixing member 1405 is drawn into the inner surface of the guide member 50 and causes jamming therein.

As illustrated in Figs. 15 and 16, the outer peripheral surface of the connection section 201 is provided with the projection portion 411. The connection device 200 further includes the connection holding body 7 that internally accommodates the adapter fixing member 1603. The connection holding body 7 is provided with the helical guide groove portion 910 which guides the projection portion 411 in a manner of gyrating around the central axis CL. Before an operation in which the projection portion 550 of the connector fixing member 1405 is guided by the helical guide following surface 55 in a gyrating manner starts, an operation in which the projection portion 411 of the connection section 201 is guided by the helical guide groove portion 910 in a gyrating manner is configured to start.

Accordingly, at the point of time the projection portion 411 of the connection section 201 enters the helical guide groove portion 910, the projection portion 550 of the connector fixing member 1405 has not yet bumped into the helical vertex portion of the helical guide following surface 55 of the guide member 50, and a gyration guiding operation for the projection portion 411 of the connection section 201 is performed prior thereto along the helical guide groove portion 910 from the leading end portion of the helical guide groove portion 910 and proceeds. When the projection portion 550 hits the helical guide following surface 55 of the guide member 50, the projection portion 550 is guided by the helical guide following surface 55 in a gyrating manner. Accordingly, the projection portion 550 can be smoothly guided by the helical guide following surface 55, and the end portions of the optical fiber cables can be reliably and smoothly connected to each other.

As illustrated in Figs. 17 and 18, the distal portion on the outer peripheral surface of the connector fixing member 1405 is provided with the guide projection 890 which guides the connector fixing member 1405 in a manner of gyrating around the central axis CL by coming into contact with the helical guide following surface 55 of the guide member 50 prior to the projection portion 550 when the connector fixing member 1405 is inserted into the guide member 50.

Accordingly, when the connector fixing member 1405 is inserted, the guide projection 890 hits the helical vertex portion of the helical guide following surface 55 prior to the projection portion 550. Therefore, it is possible to induce rotation along the helical guide following surface 55 of the guide member 50. That is, before the guiding projection portion 550 hits the helical vertex portion, the guide projection 890 bumps into the helical vertex portion. Therefore, the projection portion 550 can avoid hitting the helical vertex portion. Accordingly, the projection portion 550 can be caused to smoothly copy the helical guide following surface 55 from the position where the projection portion 550 has avoided the helical vertex portion of the helical guide following surface 55.

The imaging apparatus according to the present invention is the optical imaging apparatus 500, for example, provided with the above-described connection device. The imaging apparatus includes the catheter unit 100 that has the connection section 201; a drive device 1 that has the connection holding body 7, emits light of a light source to a target through the first optical fiber cable FC1 and the second optical fiber cable FC2, and obtains return light from the target through the first optical fiber cable FC1 and the second optical fiber cable FC2; and the operation control apparatus 501 that forms an image of the target in accordance with a signal which is generated based on the return light and is sent from the drive device 1.

Accordingly, in the optical imaging apparatus 500 which is an example of the imaging apparatus provided with the connection device according to the embodiment of the present invention, when the connector fixing member 1405 is inserted into the guide member 50 in order to connect the end portion of the first optical fiber cable FC1 and the end portion of the second optical fiber cable FC2 to each other, the projection portion 550 of the connector fixing member 1405 can be guided in a manner of gyrating along the helical guide following surface 55 formed in one direction in the guide member 50 without confusing the gyratory direction. That is, the projection portion 550 of the connector fixing member 1405 can be reliably guided in a manner of gyrating in only one direction along the helical guide following surface 55 formed in one direction in the guide member 50. Accordingly, in the present invention, the connector fixing member 1405 and the guide member 50 can be smoothly coupled to each other, and the optical fiber cables can be reliably connected to each other while jamming is prevented from occurring in the connection device.

### (Fifth Embodiment)

The connection device in each of the above-described embodiments and the imaging apparatus provided with the connection device are the optical coherence tomography apparatuses 500 (OCT) forming an image by using optical signals.

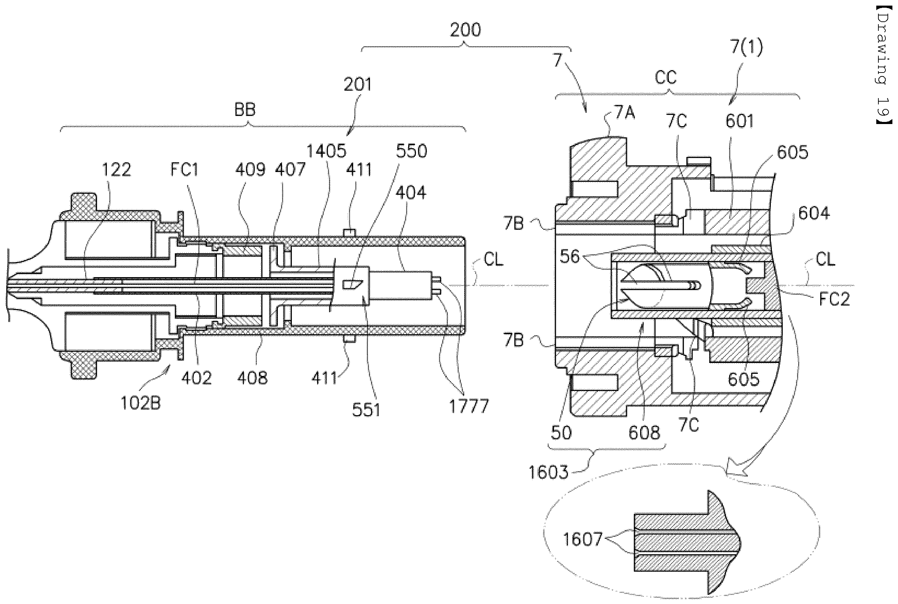

Here, as illustrated in Fig. 19, the connection device according to a fifth embodiment of the present invention is an intravascular ultrasound imaging system which forms an image by using ultrasound signals different from the optical coherence tomography apparatus 500 (OCT).

In the optical coherence tomography apparatus 500 illustrated in Fig. 3, the light transmitting and receiving unit 121 which transmits and receives measurement light is disposed inside the catheter sheath 101, and a lens is simply disposed at a position near the tube 103 ahead of the light transmitting and receiving unit 121. The light transmitting and receiving unit 121 only transmits light returned from a target object to the first optical fiber cable FC1.

In contrast, in the connection device according to the fifth embodiment of the present invention and the intravascular ultrasound imaging system serving as the imaging apparatus provided with the connection device, in place of the light transmitting and receiving unit 121, an acoustic wave transmitting and receiving unit 1121 formed of a piezoelectric material is disposed, and a transducer generating ultrasound waves is disposed in the acoustic wave transmitting and receiving unit 1121. An ultrasound signal which is transmitted and received by the acoustic wave transmitting and receiving unit 1121 is transmitted as an electrical signal through conductive cables by performing amplification of the signal, logarithmic conversion, wave detection, and the like.

As illustrated in Fig. 19, an end portion 1777 of the conductive cable serving as the first information transmission cable and an end portion 1607 of the conductive cable serving as the second information transmission cable positioned on the central axis CL of the catheter connection holding body 7 can be connected to each other by being brought into contact with each other. In a case of the intravascular ultrasound imaging system, the cable has two or more terminals. Any set of the connection terminals of the cables may be connected to each other.

The fifth embodiment is not also limited to being provided with only one guide groove portion (referred to as the slot also) 56, and the plurality of guide groove portions 56 may be provided in the guide member 50.

The slope angle BD of one helical guide following surface 55 in the illustrated example in Fig. 8 is set to 15 degrees as an example. However, without being limited thereto, for example, the slope angle BD may be set to 30 degrees.

### Reference Signs List

1...SCANNER & PULL-BACK UNIT (MOTOR DRIVE UNIT, DRIVE DEVICE)
100...CATHETER UNIT
7...CATHETER CONNECTION HOLDING BODY (CONNECTION HOLDING BODY)
50...GUIDE MEMBER
55...HELICAL GUIDE FOLLOWING SURFACE
200... CONNECTION DEVICE
201...CONNECTION SECTION
411...PROJECTION PORTION
500...OPTICAL COHERENCE TOMOGRAPHY APPARATUS (EXAMPLE OF IMAGING APPARATUS)
502...SIGNAL CABLE
501...OPERATION CONTROL APPARATUS
503...MAIN BODY CONTROL UNIT
550...PROJECTION PORTION
607...END PORTION OF SECOND OPTICAL FIBER CABLE (FC2: EXAMPLE OF SECOND INFORMATION TRANSMISSION CABLE)
608...PROTECTIVE TUBE
777...END PORTION OF FIRST OPTICAL FIBER CABLE (FC1: EXAMPLE OF FIRST INFORMATION TRANSMISSION CABLE)
880...PROJECTION PORTION
890...GUIDE PROJECTION
910...GUIDE GROOVE PORTION
1405...CONNECTOR FIXING MEMBER
1603...ADAPTER FIXING MEMBER
FC1...FIRST OPTICAL FIBER CABLE (EXAMPLE OF FIRST INFORMATION TRANSMISSION CABLE)
FC2...SECOND OPTICAL FIBER CABLE (EXAMPLE OF SECOND INFORMATION TRANSMISSION CABLE)
CL...CENTRAL AXIS

## Claims

1. A connection device (200) which connects an end portion of a first information transmission cable (FC1) and an end portion of a second information transmission cable (FC2) to each other, the connection device (200) comprising:
a connection section (201) that holds a tubular connector fixing member (1405) holding the first information transmission cable (FC1) and having a projection portion (550) on an outer peripheral surface; and
a tubular adapter fixing member (1603) that holds the second information transmission cable (FC2) and connects the end portion of the first information transmission cable (FC1) and the end portion of the second information transmission cable (FC2) to each other in accordance with insertion of the connector fixing member (1405),
wherein the adapter fixing member (1603) internally has a guide member (50) being a cylindrical member,
wherein the guide member (50) is provided with a guide groove portion (56) formed so as to be parallel to a central axis (CL) of the first information transmission cable (FC1) and the second information transmission cable (FC2) and a helical guide following surface (55) smoothly formed at a predetermined slope angle (BD) in only one direction around the central axis (CL) from a helical vertex portion (55A) to a helical rear end portion (55B) in order to perform an operation in which the projection portion (550) of the connector fixing member (1405) is guided in a manner of gyrating around the central axis (CL), when the connector fixing member (1405) is inserted, **characterized in that** the helical vertex portion (55A) and the guide groove portion (56) are adjacent to each other, the helical rear end portion (55B) and the guide groove portion (56) are also adjacent to each other, and the guide groove portion (56) is positioned between the helical vertex portion (55A) and the helical rear end portion (55B).

2. The connection device (200) according to Claim 1,
wherein an inner surface of the adapter fixing member (1603) is provided with a slope restraining projection portion (880) which restrains the connector fixing member (1405) from sloping with respect to the adapter fixing member (1603) when the connector fixing member (1405) is inserted into the guide member (50).

3. The connection device (200) according to Claim 1 or 2,
wherein an outer peripheral surface of the connection section (201) is provided with a projection portion (411),
wherein the connection device (200) further comprises:
a connection holding body (7) that internally accommodates the adapter fixing member (1603),
wherein the connection holding body (7) is provided with a helical guide groove portion (910) which guides the projection portion (411) in a manner of gyrating around the central axis (CL), and
wherein before an operation in which the projection portion (550) of the connector fixing member (1405) is guided by the helical guide following surface (55) in a gyrating manner starts, an operation in which the projection portion (411) of the connection section (201) is guided by the helical guide groove portion (910) in a gyrating manner is configured to start.

4. The connection device (200) according to any one of Claims 1 to 3,
wherein a distal portion on the outer peripheral surface of the connector fixing member (1405) is provided with a guide projection (890) which guides the connector fixing member (1405) in a manner of gyrating around the central axis (CL) by coming into contact with the helical guide following surface (55) of the guide member (50) prior to the projection portion (550) when the connector fixing member (1405) is inserted into the guide member (50).

5. The connection device (200) according to any one of Claims 1 to 4,
wherein the first information transmission cable (FC1) has an imaging core, and the imaging core generates an optical signal or an acoustic signal.

6. An imaging apparatus provided with the connection device (200) according to any one of Claims 1 to 5, the imaging apparatus comprising:
a catheter unit (100) that has a connection section (201);
a drive device (1) that has a connection holding body (7), emits a signal to a target through a first information transmission cable (FC1) and a second information transmission cable (FC2), and obtains a signal from the target through the first information transmission cable (FC1) and the second information transmission cable (FC2); and
an operation control apparatus (501) that forms an image of the target in accordance with a signal which is generated based on the signal from the target and is sent from the drive device (1).

## Patentansprüche

1. Verbindungsvorrichtung (200), die einen Endabschnitt eines ersten Informationsübertragungskabels (FC1) und einen Endabschnitt eines zweiten Informationsübertragungskabels (FC2) miteinander verbindet, wobei die Verbindungsvorrichtung (200) umfasst:
einen Verbindungsabschnitt (201), der ein rohrförmiges Verbinderbefestigungselement (1405) aufnimmt, welches das erste Informationsübertragungskabel (FC1) aufnimmt und einen Vorsprungsabschnitt (550) an einer Außenumfangsfläche aufweist; und
ein rohrförmiges Adapterbefestigungselement (1603), welches das zweite Informationsübertragungskabel (FC2) aufnimmt und den Endabschnitt des ersten Informationsübertragungskabels (FC1) und den Endabschnitt des zweiten Informationsübertragungskabels (FC2) in Übereinstimmung dem Einsetzen des Verbinderbefestigungselements (1405) miteinander verbindet,
wobei das Adapterbefestigungselement (1603) im Inneren ein Führungselement (50) aufweist, das ein zylindrisches Element ist,
wobei das Führungselement (50) mit einem Führungsnutabschnitt (56), der so ausgebildet ist, dass er parallel zu einer zentralen Achse (CL) des ersten Informationsübertragungskabels (FC1) und des zweiten Informationsübertragungskabels (FC2) verläuft, und mit einer schraubenförmigen Führungsfolgefläche (55) versehen ist, die in nur einer Richtung um die zentrale Achse (CL) von einem schraubenförmigen Scheitelabschnitt (55A) zu einem schraubenförmigen hinteren Endabschnitt (55B) gleichmäßig mit einem vorbestimmten Neigungswinkel (BD) ausgebildet ist, um eine Betätigung auszuführen, bei welcher der Vorsprungsabschnitt (550) des Verbinderbefestigungselements (1405) in einer Weise geführt wird, dass er um die zentrale Achse (CL) kreiselt, wenn das Verbinderbefestigungselement (1405) eingeführt wird,
**dadurch gekennzeichnet, dass** der schraubenförmige Scheitelabschnitt (55A) und der Führungsnutabschnitt (56) einander benachbart sind, wobei der schraubenförmige hintere Endabschnitt (55B) und der Führungsnutabschnitt (56) ebenfalls einander benachbart sind, und der Führungsnutabschnitt (56) zwischen dem schraubenförmigen Scheitelabschnitt (55A) und dem schraubenförmigen hinteren Endabschnitt (55B) angeordnet ist.

2. Verbindungsvorrichtung (200) nach Anspruch 1,
wobei eine Innenfläche des Adapterbefestigungselements (1603) mit einem Neigungsbegrenzungsvorsprung (880) versehen ist, der das Verbinderbefestigungselement (1405) daran hindert, sich in Bezug auf das Adapterbefestigungselement (1603) zu neigen, wenn das Verbinderbefestigungselement (1405) in das Führungselement (50) eingeführt wird.

3. Verbindungsvorrichtung (200) nach Anspruch 1 oder 2,
wobei eine äußere Umfangsfläche des Verbindungsabschnitts (201) mit einem vorstehenden Abschnitt (411) versehen ist,
wobei die Verbindungsvorrichtung (200) ferner umfasst:
einen Verbindungsaufnahmekörper (7), der im Inneren das Adapterbefestigungselement (1603) aufnimmt,
wobei der Verbindungsaufnahmekörper (7) mit einem schraubenförmigen Führungsnutabschnitt (910) versehen ist, welcher den Vorsprungsabschnitt (411) in einer Art und Weise führt, dass er um die zentrale Achse (CL) kreiselt, und
wobei vor dem Beginn eines Arbeitsvorgangs, bei welchem der Vorsprungsabschnitt (550) des Verbinderbefestigungselements (1405) durch die schraubenförmige Führungsfolgefläche (55) in einer kreiselnden Art und Weise geführt wird, ein Arbeitsvorgang, bei welchem der Vorsprungsabschnitt (411) des Verbindungsabschnitts (201) durch den schraubenförmigen Führungsnutabschnitt (910) in einer kreiselnden Art und Weise geführt wird, so konfiguriert ist, dass er beginnt.

4. Verbindungsvorrichtung (200) nach einem der Ansprüche 1 bis 3,
wobei ein distaler Abschnitt an der äußeren Umfangsfläche des Verbinderbefestigungselements (1405) mit einem Führungsvorsprung (890) versehen ist, der das Verbinderbefestigungselement (1405) in einer Art und Weise führt, dass es um die zentrale Achse (CL) kreiselt, indem es mit der schraubenförmigen Führungsfolgefläche (55) des Führungselements (50) vor dem Vorsprungsabschnitt (550) in Kontakt kommt, wenn das Verbinderbefestigungselement (1405) in das Führungselement (50) eingeführt wird.

5. Verbindungsvorrichtung (200) nach einem der Ansprüche 1 bis 4,
wobei das erste Informationsübertragungskabel (FC1) einen Bildgebungskern aufweist, und der Bildgebungskern ein optisches Signal oder ein akustisches Signal erzeugt.

6. Bildgebungsvorrichtung mit der Verbindungsvorrichtung (200) nach einem der Ansprüche 1 bis 5, wobei die Bildgebungsvorrichtung umfasst:
eine Kathetereinheit (100), die einen Verbindungsabschnitt (201) aufweist;
eine Antriebsvorrichtung (1), die einen Verbindungsaufnahmekörper (7) aufweist, ein Signal zu einem Ziel über ein erstes Informationsübertragungskabel (FC1) und ein zweites Informationsübertragungskabel (FC2) aussendet und ein Signal von dem Ziel über das erste Informationsübertragungskabel (FC1) und das zweite Informationsübertragungskabel (FC2) empfängt; und
eine Betriebssteuerungsvorrichtung (501), die ein Bild des Ziels in Übereinstimmung mit einem Signal erzeugt, das auf der Grundlage des Signals von dem Ziel erzeugt wird und von der Antriebsvorrichtung (1) gesendet wird.

## Revendications

1. Dispositif de raccordement (200) qui raccorde une partie d'extrémité d'un premier câble de transmission d'informations (FC1) et une partie d'extrémité d'un deuxième câble de transmission d'informations (FC2) l'une à l'autre, le dispositif de raccordement (200) comprenant :
une section de raccordement (201) qui maintient un élément de fixation de connecteur tubulaire (1405) maintenant le premier câble de transmission d'informations (FC1) et présentant une partie faisant saillie (550) sur une surface périphérique extérieure ; et
un élément de fixation d'adaptateur tubulaire (1603) qui maintient le deuxième câble de transmission d'informations (FC2) et raccorde la partie d'extrémité du premier câble de transmission d'informations (FC1) et la partie d'extrémité du deuxième câble de transmission d'informations (FC2) l'une à l'autre conformément à l'insertion de l'élément de fixation de connecteur (1405), dans lequel l'élément de fixation d'adaptateur (1603) présente en interne un élément de guidage (50), qui est un élément cylindrique,
dans lequel l'élément de guidage (50) est fourni avec une partie de rainure de guidage (56) formée de manière à être parallèle à un axe central (CL) du premier câble de transmission d'informations (FC1) et du deuxième câble de transmission d'informations (FC2) et avec une surface de suivi de guidage hélicoïdale (55) formée régulièrement selon un angle de pente prédéterminé (BD) dans seulement une direction autour de l'axe central (CL) depuis une partie de sommet hélicoïdale (55A) vers une partie d'extrémité arrière hélicoïdale (55B) pour réaliser une opération, dans laquelle la partie faisant saillie (550) de l'élément de fixation de connecteur (1405) est guidée de manière à tourner autour de l'axe central (CL), lorsque l'élément de fixation de connecteur (1405) est inséré,
**caractérisé en ce que** la partie de sommet hélicoïdale (55A) et la partie de rainure de guidage (56) sont adjacentes l'une à l'autre, la partie d'extrémité arrière hélicoïdale (55B) et la partie de rainure de guidage (56) sont également adjacentes l'une à l'autre, et la partie de rainure de guidage (56) est positionnée entre la partie de sommet hélicoïdale (55A) et la partie d'extrémité arrière hélicoïdale (55B).

2. Dispositif de raccordement (200) selon la revendication 1,
dans lequel une surface interne de l'élément de fixation d'adaptateur (1603) est fournie avec une partie faisant saillie de retenue de pente (880), qui retient l'élément de fixation de connecteur (1405) l'empêchant de descendre par rapport à l'élément de fixation d'adaptateur (1603) lorsque l'élément de fixation de connecteur (1405) est inséré dans l'élément de guidage (50).

3. Dispositif de raccordement (200) selon la revendication 1 ou 2,
dans lequel une surface périphérique extérieure de la section de raccordement (201) est fournie avec une partie faisant saillie (411),
dans lequel le dispositif de raccordement (200) comprend en outre :
un corps de maintien de raccordement (7) qui loge en interne l'élément de fixation d'adaptateur (1603),
dans lequel le corps de maintien de raccordement (7) est fourni avec une partie de rainure de guidage hélicoïdale (910), qui guide la partie faisant saillie (411) de manière à tourner autour de l'axe central (CL), et
dans lequel avant une opération, dans laquelle la partie faisant saillie (550) de l'élément de fixation de connecteur (1405) est guidée par la surface de suivi de guidage hélicoïdale (55) de manière rotative ne débute, une opération, dans laquelle la partie faisant saillie (411) de la section de raccordement (201) est guidée par la partie de rainure de guidage hélicoïdale (910) de manière rotative, est configurée pour débuter.

4. Dispositif de raccordement (200) selon l'une quelconque des revendications 1 à 3,
dans lequel une partie distale sur la surface périphérique extérieure de l'élément de fixation de connecteur (1405) est fournie avec une saillie de guidage (890), qui guide l'élément de fixation de connecteur (1405) de manière à tourner autour de l'axe central (CL) en venant en contact avec la surface de suivi de guidage hélicoïdale (55) de l'élément de guidage (50) avant la partie faisant saillie (550) lorsque l'élément de fixation de connecteur (1405) est inséré dans l'élément de guidage (50).

5. Dispositif de raccordement (200) selon l'une quelconque des revendications 1 à 4,
dans lequel le premier câble de transmission d'informations (FC1) présente un noyau d'imagerie, et le noyau d'imagerie génère un signal optique ou un signal acoustique.

6. Appareil d'imagerie fourni avec le dispositif de raccordement (200) selon l'une quelconque des revendications 1 à 5, l'appareil d'imagerie comprenant :
une unité cathéter (100) qui présente une section de raccordement (201) ;
un dispositif d'entraînement (1) qui présente un corps de maintien de raccordement (7), émet un signal vers une cible par l'intermédiaire d'un premier câble de transmission d'informations (FC1) et d'un deuxième câble de transmission d'informations (FC2), et obtient un signal provenant de la cible par l'intermédiaire du premier câble de transmission d'informations (FC1) et du deuxième câble de transmission d'informations (FC2) ; et
un appareil de commande d'opération (501), qui forme une image de la cible conformément à un signal qui est généré sur la base du signal provenant de la cible et est envoyé depuis le dispositif d'entraînement (1).
